# EUROPEAN PATENT SPECIFICATION

(11) **EP 0 804 215 B1**
(45) Date of publication and mention of the grant of the patent: **28.11.2001**
(21) Application number: 94925866.9
(22) Date of filing: 11.08.1994
(51) Int. Cl.: C07K 1/107, C07K 7/02

(54) **TEMPLATE ASSEMBLED SYNTHETIC PROTEIN**
ANHAND EINER VORLAGE AUFGEBAUTES SYNTHETISCHES PROTEIN
PROTEINE SYNTHETIQUE ASSEMBLEE PAR MATRICE

(30) Priority: 11.08.1993 US 105369
(43) Date of publication of application: 05.11.1997
(73) Proprietor: THE SCRIPPS RESEARCH INSTITUTE, La Jolla, CA 92037 (US)
(72) Inventor: DAWSON, Philip E., La Jolla, CA 92037 (US); KENT, Stephen B.H., La Jolla, CA 92037 (US)
(74) Representative: Hedley, Nicholas James Matthew
(86) International application number: US9409165
(87) International publication number: WO9504543

(56) References cited:
- US-A- 5 066 716
- P.DAWSON AND S. KENT: "Convenient total synthesis of a 4-helix TASP molecule by chemoselective ligation" JOURNAL OF THE AMERICAN CHEMICAL SOCIETY., vol. 1993, no. 115, 11 August 1993, DC US, pages 7263-7266, XP002062451
- M.MUTTER ET AL.: "Template assembled synthetic proteins with four-helix-bundle topology" JOURNAL OF THE AMERICAN CHEMICAL SOCIETY., vol. 114, 1992, DC US, pages 1463-1470, XP002062452
- M. SCHNÖLZER AND S. KENT: "Constructing proteins by dovetailing unprotected synthetic peptides" SCIENCE, vol. 256, 1992, pages 221-225, XP002062453
- ANGEWANTE CHEMIE INTERNATIONAL EDITION IN ENGLISH, Vol. 28, No. 5, issued May 1989, M. MUTTER et al., "A Chemical Approach to Protein Design- Template-Assembled Synthetic Proteins (TASP)", pages 535-554.

## Description

### Technical Field:

The invention relates to the synthesis of branched or dendritic peptides. More particularly, the invention relates to the synthesis of template assembled synthetic protein (TASP) molecules using chemoselective ligation procedures and to the genus of TASP's which employ dendritic linkage units having the structure ψ(CO-S-CH₂-CO-NH).

### Background Art:

Since the time of Emil Fischer, it has been a goal of organic chemists to design and chemically synthesize proteins. In recent years there has been a growing interest in the de *novo* design of proteins, particularly helix-bundle proteins, and their production by means of chemical synthesis or recDNA-expression. Examples of helix-bundle proteins designed *de novo* are provided by M. Hecht et al. (Science, **1990**, vol. *249,* pages 884-891), by L. Regan et al. *(Science,* **1988**, vol. *241,* pages 976-978), by W. F. DeGrado et al. *(Science,* **1989**, vol. *243*, pages 622-628), and by N.E. Zhou et al. *(Biochemistry,* **1992**, vol. *31*, pages 5739-5746).

In some cases, unexpected results have been obtained in which the experimentally observed structure of synthetic helix bundles has been different than the intended structure because of the uncontrolled nature of non-covalent intermolecular association, e.g., see B. Lovejoy et al. *(Science,* **1993**, vol. *259,* pages 1288-1293). To avoid such problems, helix bundle proteins have been made by the preparation of covalent arrays linked through porphyrin molecules or through metal chelate complexes. Preliminary evidence indicates that the expected structures have been achieved, e.g., see T. Sasaki et al. (*J. Am. Chem. Soc.,* **1989**, vol. *111*, pages 380-381) and R. M. Ghadiri et al. (*J. Am. Chem. Soc.,* **1992**, vol. *114*, pages 4000-4002).

An alternative, earlier approach to the preparation of covalent peptide arrays of predetermined secondary and tertiary structure is the "template assembled synthetic protein" (TASP) concept, disclosed by M. Mutter (*Peptides-Chemistry and Biology, Proceedings of the 10th American Peptide Symposium;* Marshall, G. R., Ed.; Escom: Leiden, **1988;** pages 349-353). A template molecule is used to covalently anchor arrays of secondary structural elements. The distinctive feature of the TASP approach is the nonlinear topology used; the molecule is made up of an array of branched polypeptides, rather than the folded linear polypeptide chain of natural proteins, e.g., M. Mutter et al., (*Angew. Chem*., Int. Ed. Engl., **1989**, vol. *28*, pages 535-554). It is anticipated that this elegant concept will have a profound effect on the *de novo* design of proteins.

However, conventional synthetic approaches for preparing TASP molecular assemblies are arduous and/or provide low yields. For example, both stepwise solid phase synthesis (SPPS) and protected segment condensation approaches have been employed with limited success. M. Mutter, M. et al. (J. Am. Chem. *Soc.,* **1992**, vol. *114*, pages 1463-1470) discloses an example of a stepwise solid phase synthesis (SPPS) of a template assembled synthetic protein. In addition, B. Dorner et al. *(Innovation and Perspectives in Solid Phase Synthesis,* Roger Epton Ed.; Intercept Limited: Andover, **1992;** pages 163-170) and by I. Ernest et al. *(Tetrahedron Lett.,* **1990**. vol. *31*, pages 4015-4018) disclose examples of the protected segment condensation approach for synthesizing template assembled synthetic proteins. Only a minimal number of TASP molecules have been produced by arduous synthetic efforts, e.g. supra and M. Mutter et al. *(Proteins,* **1989**, vol. *5*, pages 13-21) and G. Tuchscherer *(Protein Science,* **1992**, vol. 1, pages 1377-1386). Despite the exquisite care with which some of these syntheses have been performed, questions still remain with respect to TASP preparations as homogeneous molecular species of defined covalent composition. A convenient, direct general preparation of these molecules in unambiguous fashion would have great utility.

Recently, M. Schnolzer introduced the chemoselective ligation of unprotected peptide segments as a route to the total chemical synthesis of protein analogs of native (i.e. linear) topology *(Science,* **1992**, vol. *256,* pages 221-225). This approach uses unique, mutually reactive functionalities, one type on each segment, to covalently assemble long chain molecules from completely unprotected peptide segments. In this way, maximal advantage is taken of our ability to synthesize, handle, purify, and characterize unprotected peptides. Solubility problems are reduced and the target molecule is produced directly in the final unprotected form.

### Disclosure of the Invention:

The invention employs a chemoselective ligation approach in the synthesis of TASP molecules. The approach used is shown in **Scheme I**. The TASP is assembled from short unprotected peptide segments, which are synthesized in straightforward fashion by standard methods and which are readily purified to high levels of homogeneity. The target 4-helix TASP molecule was designed on the basis of the work of Mutter (Tetrahedron, **1988**, vol. 44, pages 771-785). The final molecule contains a total of four copies of the helix-forming peptide 1, one copy attached to the side chain of each of four lysine residues in a template molecule. The template molecule **2** contains a central Gly-Pro sequence to facilitate the formation of a reverse-turn structure, and to thus promote the association of the helix-forming peptides.

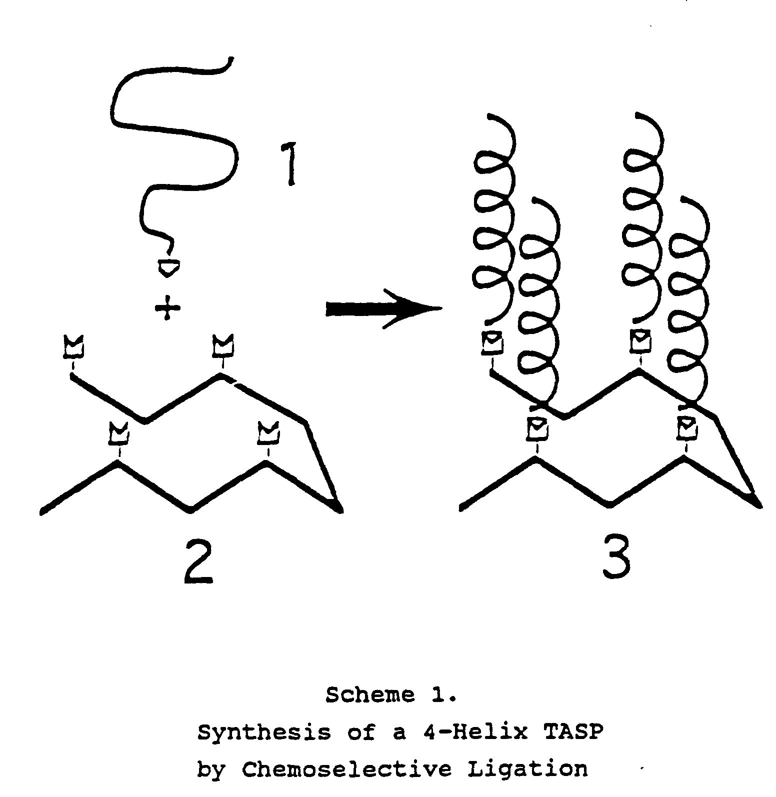

### Brief Description of the Drawings:

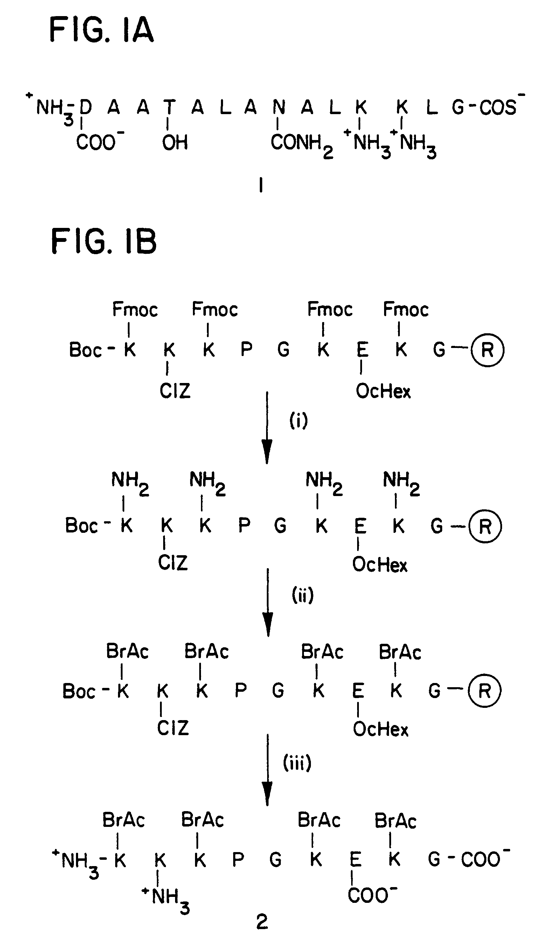

Figures 1 (A) and (B) illustrate examples of unprotected synthetic peptides for use in the TASP synthesis.
Figure 1 (A) illustrates a fourteen residue *pro*-helix peptide-^{α} thiocarboxylate 1, prepared by SPPS on a thioester resin.
Figure 1 (B) illustrates a nine residue peptide template 2, prepared by SPPS using N^{α}Boc protection and both base- and acid-labile side chain protection to allow differential modification of the side chains. Step (i) employs 50% piperidine in DMF; step (ii) employs bromoacetic anhydride in DCM; step (iii) employs HF/10% *p*-cresol, 1 hour, 0°C. Details are provided in the Materials and Methods section.
Figures 2 (A) and (B) illustrate a reaction of the pro-helix peptide-^{α}thiocarboxylate **1** with the (BrAc) template peptide **2**, in aqueous solution at pH 5.

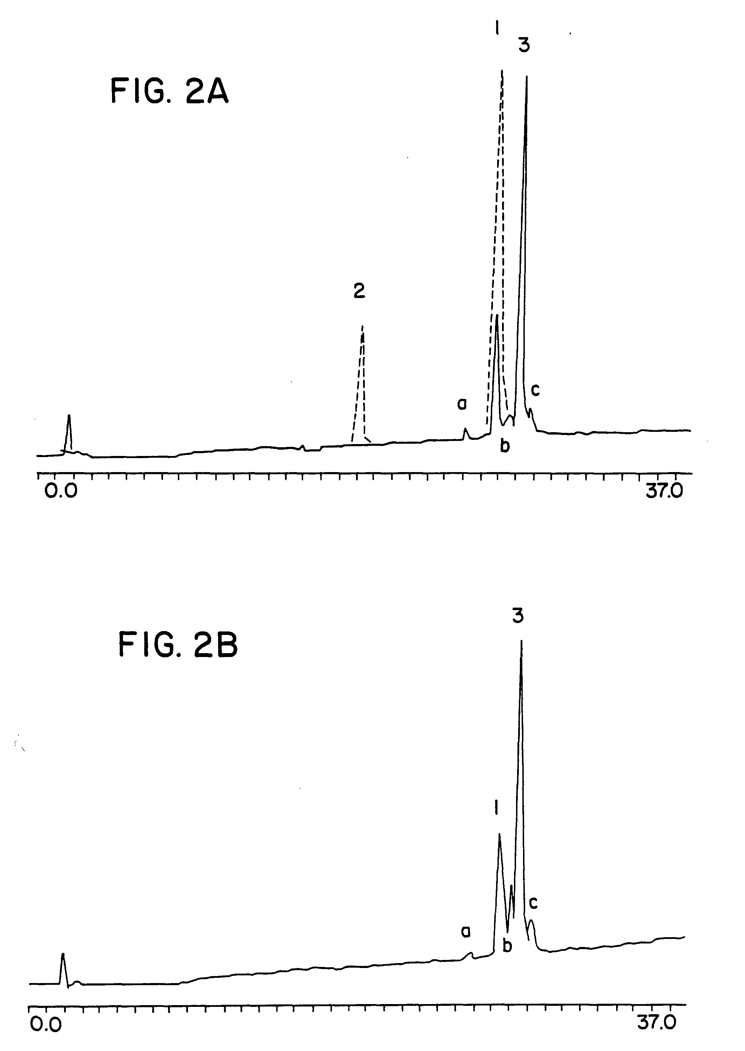

Figure 2 (A) illustrates an analytical HPLC of the reaction mixture after 5 hours of reaction. Positions and original amounts of the reactants, i.e., the branch peptide **(1)** and the template peptide **(2),** are indicated by dashed-line peaks. The product 4-helix TASP **(3)** is indicated by a solid line. Minor components were identified by ionspray mass spectrometry (MS). Peak **"a"** is a pro-helix peptide-^{α}carboxylate formed by hydrolysis of the ^{α}thiocarboyxlate. Peak **"b"** is a (*pro*-helix)₃(BrAc)₁ template. Peak **"c"** is a dimer (*pro*-helix-^{α}COS-)₂ formed by atmospheric oxidation.
Figure 2 (B) illustrates an analytical HPLC of the reaction mixture after 70 min. Products are labeled and identified as above.

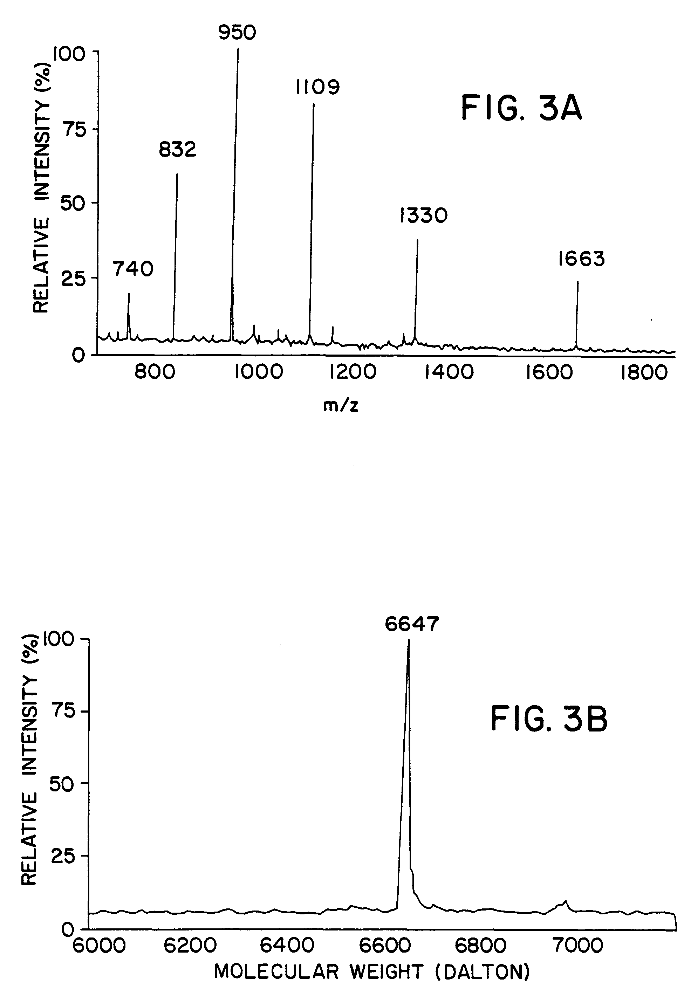

Figure 3 (A) illustrates the ionspray mass spectrometry (MS) of HPLC-purified peak **3**. Raw data is shown. The multiple charge states all arise from protonation of a single molecular species having a molecular weight of 6647 Daltons. No significant other species were detected in the HPLC fraction.
Figure 3 (B) illustrates a reconstruction of the total raw MS data to a single charge state.

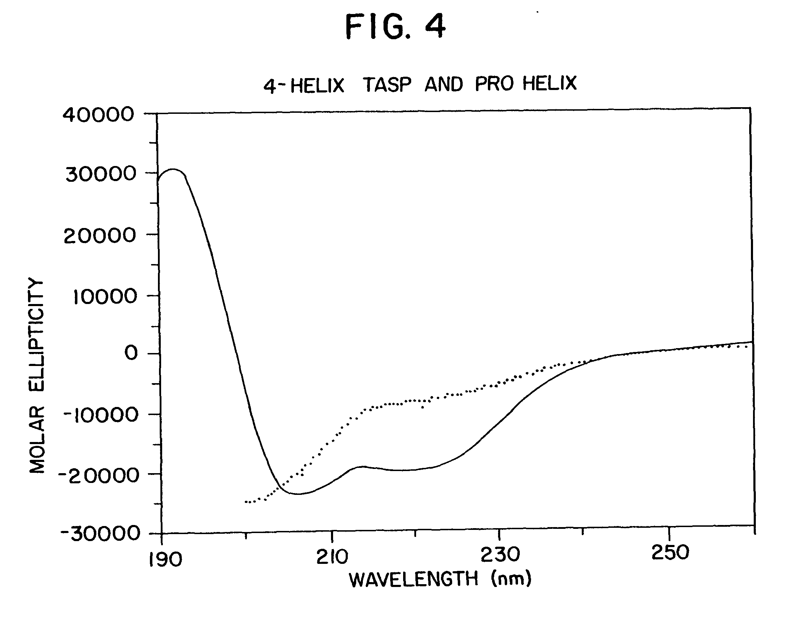

Figure 4 illustrates the circular dichroism spectrum in water of the HPLC-purified 4-helix TASP product **3** (solid line). Characteristic minima at 220nm and 208 nm and a maximum at 193 nm indicate high helical content for the ligated TASP. Under identical conditions the *pro*-helix peptide **1** (dotted line) gave a weak featureless spectrum with no indication of helical structure.

### Best Mode for Carrying Out the Invention:

The invention is a template assembled synthetic protein (TASP) employing a dendritic linkage unit with a structure ψ(CO-S-CH₂-CO-NH). More particularly, the TASP includes at least one template peptide and one branch peptide joined to one another using the dendritic linkage unit indicated above. The template peptide includes at least one amino acid residue with a template side chain bearing an amino group, preferably a lysine group. The template peptide is coupled to the dendritic linkage unit via the lysine or other side chain bearing an amino group. The branch peptide is coupled to the dendritic linkage unit via its C-terminal. An important aspect of the invention is the chemoselective linkage between the template peptide and the branch peptide, i.e., the structure ψ(CO-S-CH₂-CO-NH) which couples the C-terminal end of the branch peptide to the template peptide via the amino group of the template side chain. The dendritic linkage unit may be schematically illustrated as follows: 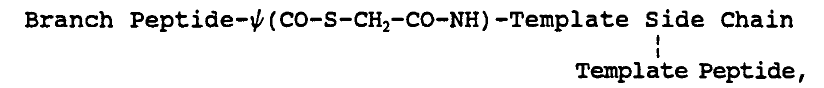 The ensemble forms a template assembled synthetic protein (TASP).

In a preferred embodiment, the branch peptide is a pro-helical peptide which, upon incorporation into the template assembled synthetic protein (TASP) and exposure to helix promoting conditions, is capable of assuming a helical configuration. A preferred branch peptide has the following sequence, viz. Seq. 1: The above branch peptide is a pro-helical peptides which, upon incorporation into a template assembled synthetic protein (TASP) and exposure to helix promoting conditions, is capable of assuming helical configurations. The TASP may include only one branch peptide or may include multiple branch peptides. A preferred TASP includes four branch peptides having Sequence 1. When multiple branch peptides are employed, each branch peptide is coupled to the template peptide via its own dendritic linkage unit.

A preferred template peptide has the following sequence, viz. Seq. 2: wherein K(Ac-) represents a lysine amino acid residue having an lysine side chain with an acetylated amino group.

The invention also contemplates the process for preparing a template assembled synthetic protein (TASP). The process comprises the following steps:
Step A: providing an unprotected template peptide including an amino acid residue having a side chain bearing an amino group bonded to a haloacetyl functionality;
Step B: providing unprotected branch peptides each bearing a ^{α}COSH moiety at its C-terminal end; and then
Step C: combining the unprotected template peptide from said Step A with the unprotected branch peptide of said Step B under nucleophilic reaction conditions for promoting a substitution reaction between the ^{α} COSH moiety of the branch peptides and the haloacetyl functionalities of the template molecule for chemoselectivly ligating the branch peptides to the template peptide and forming the template assembled synthetic protein (TASP).

In a preferred mode of the process, the haloacetyl functionality of the template peptide of Step A is a bromoacetyl group attached to the amino group of a lysine side chain. In Step C, the dendritic linkage unit is formed when the ^{α}COSH moiety of the branch peptides and the bromoacetyl group of the template molecule undergo a nucleophilic substitution reaction to create a dendritic linkage unit having the following structure, viz.:

ψ(CO-S-CH₂-CO-NH).

A preferred mode of the process for preparing a TASP may also include an additional step after completion of the assembly for exposing the reaction product to helix promoting conditions for changing the conformation of the branch peptides into a helical conformation.

### Example:

The synthetic peptide **1**, intended to form a 13 residue amphipathic helix under suitable helix-promoting conditions, was prepared with an additional C-terminal -Gly^{α}COSH residue **(Figure 1A)**. This pro-helix peptide was reacted with the template peptide **2** containing four lysine side chains modified to contain bromoacetyl moieties **(Figure 1B)**. Peptides were chemically synthesized by manual stepwise solid phase methods according to published procedures, e.g., S. B. Kent (*Ann Rev Biochem*., **1988**, vol. *57*, pages 957-989) and M. Schnolzer et al. (*Int. J. Peptide Protein Res.,* **1992**, vol. *40*, pages 180-193). Acidolytic cleavage of the pro-helix peptide from a thioester resin generates the peptide-^{α} COSH **1**, e.g., J. Blake (*Int. J. Peptide Protein Res.,* **1981**, vol. *17*, pages 273-274) and D. Yamashiro (*J. Int. J. Peptide Protein Res.,* **1988,** vol. *31,* pages 322). A combination of base-labile and acid-labile protecting groups was used to generate the template molecule **2** with four of the five lysine sidechains modified with bromoacetyl groups (Figure 1B), e.g., R. A. Robey et al. (*Anal. Biochem.,* **1989**, vol. *177,* pages 373-377). The range of functionalities present in the two peptides is shown in **Figure 1.**

The unprotected peptides were simply ligated in the desired fashion by nucleophilic reaction between the ^{α}COSH moiety of the pro-helix peptide **1** and the bromoacetyl functionalities of the template molecule **2**. The reaction proceeded cleanly over several hours at ambient temperature in aqueous buffer at pH 5.0 to give a near-quantitative yield of the 4-helix TASP molecule **3 (Figure 2A)**. The reaction was monitored by direct ionspray MS of the reaction mixture and by analytical HPLC, e.g., M. Schnolzer *(Anal. Biochem.,* **1992,** vol. 204, pages 335-343). Although salts generally interfere with the evaporative ionization process, the dilute NH₄OAc buffer used was compatible with direct ionspray MS of synthetic peptides. An excess of the peptide-^{α}COSH component **1** was used, and the final reaction mixture was fully depleted of the (BrAc)₄ template molecule **2**. The ligation proceeded very quickly as monitored by HPLC. After just 70 minutes, the reaction had progressed almost to completion **(Figure 2B)**. In addition to the target 4-helix TASP **3**, the only other detectable components were residual excess *pro*-helix-^{α}COSH **1**, residual amounts of (*pro*-helix)₃(BrAc)₁ template, and trace amounts of *pro*-helix-^{α}COOH and the dimer (*pro*-helix-^{α} COS-)₂. It is of some interest that the only intermediate reaction product detected **(Figure 2B)** was the (*pro*-helix)₃(BrAc)₁template.

The desired product was readily purified by HPLC and was lyophilized to yield a white solid. This was characterized by ionspray mass spectrometry **(Figure 3)** and was found to be the target 4-helix TASP 3 in high purity, and with the expected mass, i.e., the observed molecular weight was 6647.1±2.8 Daltons, while the calculated molecular weight for a monoisotopic C₂₈₈H₅₀₃N₈₂O₈₈S₄ is 6645.6 and the calculated molecular weight for C₂₈₈H₅₀₃N₈₂O₈₈S₄ having an average isotope composition is 6649.9. The ligated 4-helix TASP 3 was stable for days at ambient temperatures in pH 5.0 10mM NH₄OAc, and was indefinitely stable at 4°C at pH 6.0. Circular dichroism spectroscopy was used to determine the secondary structure of the ligated TASP molecule 3 in water. The molecule was highly helical **(Figure 4).** Control studies of peptide 1 under identical conditions showed no helical content whatever. These conformational properties were similar to those observed for the closely related 4-helix TASP prepared by conventional means (supra). Ionspray MS under native conditions¹⁷ showed that the 4-helix TASP occurred as a monomeric species, e.g., M. Baca et al. (*J. Am. Chem*. *Soc.,* **1992**, vol. *114*, pages 3992-3993).

The overwhelming feature of this synthetic approach is its simplicity. The ligation occurred rapidly in aqueous solution and essentially no other reaction products were detected. The product was readily purified to give exceptionally homogeneous material with a mass consistent with the proposed covalent structure. The observed helical secondary structure was consistent with the formation of the target 4-helix TASP molecule **3**.

The major advantages of the chemoselective ligation approach include the elimination of microheterogeneity in the final product and the generality of the approach. Peptides simultaneously built up on the template by stepwise solid-phase synthesis yield very heterogeneous crude products. Mutter and co-workers have demonstrated the use of stringent chromatographic techniques to increase the purity of the final product (supra), but these protocols have not been applied to the larger and more hydrophobic ion channel assemblies prepared by Montal and co-workers (*Proc. Natl Acad. Sci. U.S.A*., **1991**, vol. *88*, pages 6418-6422). Segment condensation of protected peptide fragments has been used to decrease heterogeneity but problems with solubility lead to extremely slow ligation reactions resulting in low yields. Recent work by DeGrado and co-workers used unprotected peptide fragments, but is of limited applicability due to the incompatibility of the ligation reaction with all functional groups, especially the ∈-amino group of lysine (*J. Am. Chem. Soc.,* **1992**, vol. *114*, pages 9656-9657).

In contrast to these approaches, the chemoselective ligation approach allows ready purification of the unprotected peptide components, which can be ligated at high concentration in a fast, clean reaction. The target compound is obtained directly in the final unprotected form and is readily purified. The method is of general applicability because the chemistry is compatible with all functional groups found in peptides and proteins (*Science*, **1992**, vol. *256*, pages 221-225). In previous work we have shown that purified unprotected peptides can be correctly ligated in solvents such as 6 M guanidine HCl or in organic/aqueous mixtures. This allows great flexibility in the selection of *pro*-helix sequences and of solvent conditions to maintain the necessary solubility for rapid reaction.

### Materials and Methods:

Analytical and semipreparative gradient HPLC was performed on a Rainin dual pump high pressure mixing system with 214 nm UV detection using Vydac C-18 analytical (5 micron, 0.46 x 15 cm) and semipreparative (10 m, 1.0 x 25 cm) columns. Analytical runs used a 0%-67% B gradient over 30 min at 1mL/min where buffer A is 0.1% TFA in H₂O and buffer B is 90% CH₃CN + 10% buffer A. Mass spectra were obtained using a Sciex API-III quadrupole ion-spray mass spectrometer. CD measurements were obtained using an Aviv 62 DS instrument. Peptide concentrations were determined by amino acid analysis after hydrolysis in 6 N HCl for 24 hours at 110°C.

**Synthesis of pro-helix peptide 1.** Except where noted, peptides were synthesized by manual stepwise solid phase methods according to published procedures, e.g., S. B. Kent (*Ann Rev Biochem*., **1988**, vol. *57*, pages 957-989) and M. Schnolzer et al. (*Int. J. Peptide Protein Res.,* **1992**, vol. *40*, pages 180-193). Coupling yields were monitored by quantitative ninhydrin. The pro-helix was synthesized on a Gly thioester resin using standard N^{a}Boc chemistry SPPS, e.g., J. Blake (*Int. J. Peptide Protein Res.,* **1981**, vol. *17,* pages 273-274) and D. Yamashiro (*J. Int. J. Peptide Protein Res.,* **1988**, vol. *31*, page 322). The peptide was deprotected and simultaneously cleaved from the resin by treatment with HF plus 2% anisole for 1 hour at 0°C. The crude peptide was taken up in neat TFA, diluted with ddH₂O to 0.5% TFA and lyophilized to remove residual anisole. The pro-helix was purified by semipreparative reversed phase HPLC (0%-67% buffer B over 60 min at 3 mL/min) and characterized by ionspray mass spectrometry, i.e., the observed molecular weight was 1372.2 ± 0.4 Daltons while the calculated molecular weight is 1371.8 daltons (monoisotopic) or 1372.6 daltons (average isotope composition).

**Synthesis of template peptide 2.** The template peptide was synthesized using a combination of N^{a}Boc-chemistry SPPS and N^{e}Fmoc lysine side chain protection on a Gly-OCH₂-Pam-resin **(Fig 1B)**. To minimize the possibility of premature Fmoc removal, a separate brief neutralization with 5% diisopropylethylamine/DMF was used, rather than *in situ* neutralization in the coupling step, e.g., M. Schnolzer et al. (*Int. J. Peptide Protein Res.,* **1992,** vol. *40,* pages 180-193). Following nine synthetic cycles, the Fmoc protecting groups on the lysine side chains were removed by two 5 minute treatments with 50% piperidine/DMF. The free e-amino groups were bromoacetylated using bromoacetic acid/DIC coupling, e.g., R. A. Robey et al. *(Anal. Biochem.,* **1989**, vol. *177,* pages 373-377). The peptide was deprotected and cleaved by HF plus 10% *p*-cresol over 1h at 0°C, using standard protocols, e.g., M. Schnolzer et al. (*Int. J. Peptide Protein Res.,* **1992**, vol. *40*, pages 180-193). The template was then purified to homogeneity by semipreparative reversed phase HPLC (25%-41% buffer B over 30 minutes at 3mL/minute) and characterized by ionspray mass spectrometry, i.e., the observed molecular weight was 1482.7 ± 0.4 Daltons while the calculated molecular weight is 1478.3 daltons (monoisotopic) or 1483.0 daltons (average isotope composition).

**Synthesis of 4-helix TASP 3.** Ligation was performed by combining 0.50 mg of the (BrAc)₄ template (Formula weight 1484 Daltons, 3.36 x 10⁻⁷ mole, 1.12 mM), and 2.75 mg pro-helix-^{α}COSH (Formula weight 1372 Daltons, 2.0 x1 0⁻⁶ mole, 6.68 mM) in 300 *µ*L of 10mM NH₄OAc aqueous buffer pH 5.0 at 23°C. Reaction was monitored by analytical reverse phase HPLC (4 *µ*L aliquots). Peaks were collected based on UV absorbance and examined by ionspray MS, e.g., M. Schnolzer (Anal. *Biochem*., **1992**, vol. *204*, pages 335-343). After 6.5 hours at 23°C, the reaction mixture was stored at 4°C. Product (170 *µ*L of reaction mixture) was purified by reversed phase HPLC (38%-54% buffer B over 30 min at 3mL/minute) and lyophilized giving 0.26 mg of pure product, theoretical yield: 1.27mg, 20.5%. The mass was determined by ionspray mass spectrometry, i.e., the observed molecular weight was 6647.1 ± 2.8 Daltons while the calculated molecular weight is 6645.6 daltons (monoisotopic) or 6649.9 daltons (average isotope composition).

**Stability.** Stability was monitored by analytical reversed phase HPLC and mass spectrometry of collected peaks. 1 mM 4-helix TASP from the ligation reaction was stored at 23°C for several days without degradation. 5 mM 4-helix TASP stored in 100 mM Phosphate buffer pH 6.0 showed no decomposition after 7 days at 4°C.

**Circular Dichroism.** Both the *pro*-helix (7.5 *µ*M) and the 4-helix TASP (1.78 *µ*M) were dissolved in doubly distilled H₂O. Measurements were taken in a 2mL cuvette with a path length of 1cm at 20.0°C, scanning from 260nm to 190nm every 0.50 nm.

### Conclusion

These results indicate the potential of the chemoselective ligation approach as a general route to the preparation of TASP-like macromolecules. Combinations of existing chemical tactics provide great versatility for the selective introduction of reactive moieties into unprotected peptide building blocks which can be used in the design and synthesis of a variety of TASP-related compounds. In particular, the thioester nucleophilic ligation chemistry described here can be combined with other ligation chemistries originally developed for rejoining fragments of proteins to generate a great diversity of nonlinear covalent molecular topologies, e.g., K. Rose et al. (*Bioconjugate Chem.,* **1991,** vol. *2,* pages 154-159) and H. Gaertner et al. (*Bioconjugate Chem.,* **1992,** vol. *3*, pages 262-268). In conjunction with modern analytical protein chemistry the chemoselective synthetic approach is a powerful adjunct to the design and study of conformations and activities of protein-related macromolecules.

## Claims

1. A template assembled synthetic protein (TASP) having a dendritic linkage unit with a structure ψ(CO-S-CH₂-CO-NH).

2. A template assembled synthetic protein (TASP) as described in claim 1 further comprising:
four dendritic linkage units having the structure ψ(CO-S-CH₂-CO-NH),
four branch peptides, each of said branch peptides having a sequence ⁺NH₃-D-A-A-T-A-L-A-N-A-L-K-K-L-G-COS-, and
a template peptide having a sequence ⁺NH₃-K(Ac-)-K(⁺NH₃)-K (Ac-) -P-G-K(Ac-)-E(COO⁻)-K (Ac-)-G-COO⁻,
said four linkage units coupling said branch peptides to the four K(Ac-) residues of said template peptide.

3. An improved template assembled synthetic protein (TASP) having a branch peptide with a C-terminal end and a template peptide including an amino acid residue with a template side chain bearing an amino group, wherein the improvement comprises:
a linkage unit having the structure ψ(CO-S-CH₂-CO-NH) coupling the C-terminal end of said branch peptide to said template peptide via the amino group of the template side chain as follows: 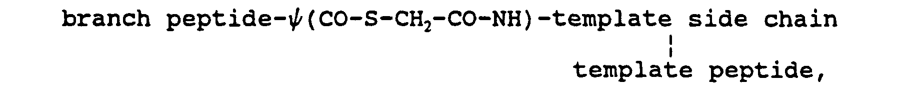
whereby the branch peptide serves as a branch upon the template peptide to form a template assembled synthetic protein (TASP).

4. An improved template assembled synthetic protein (TASP) as described in claim 3 wherein:
the template side chain of said template peptide is a lysine side chain corresponding to a lysine amino acid residue.

5. An improved template assembled synthetic protein (TASP) as described in claim 3 wherein:
said branch peptide is a pro-helical peptide which, upon incorporation into the template assembled synthetic protein (TASP) and exposure to helix promoting conditions, is capable of assuming a helical configuration.

6. An improved template assembled synthetic protein (TASP) as described in claim 3 wherein the branch peptide includes a first branch peptide and a second branch peptide, each with a C-terminal end, and the template peptide includes a first amino acid residue with a first template side chain bearing an amino group and a second amino acid residue with a second template side chain bearing an amino group, wherein the improvement further comprises:
said linkage unit including a first linkage unit and a second linkage unit, each linkage unit having the structure ψ(CO-S-CH₂-CO-NH), the first linkage unit serving to couple the C-terminal end of said first branch peptide to said template peptide via the first template side chain and the second linkage unit serving to couple the C-terminal end of said second branch peptide to said template peptide via the second template side chain,
whereby the first and second branch peptides serve as first and second branches upon the template peptide to form a template assembled synthetic protein (TASP) having two branches.

7. An improved template assembled synthetic protein (TASP) as described in claim 6 wherein:
both said first and second branch peptides are pro-helical peptides which, upon incorporation into the template assembled synthetic protein (TASP) and exposure to helix promoting conditions, are capable of assuming helical configurations.

8. A process for preparing a template assembled synthetic protein (TASP) comprising the following steps:
Step A: providing a template peptide including an amino acid residue having a template side chain bearing an amino group bonded to a haloacetyl functionality;
Step B: providing one or more branch peptides, each of said branch peptides including a C-terminal end bearing an ^{α}COSH moiety; and then
Step C: combining the template peptide from said Step A with one or more of the branch peptides of said Step B under nucleophilic reaction conditions for promoting a substitution reaction between the ^{α}COSH moiety of the branch peptides and the haloacetyl functionality of the template molecule for chemoselectivly ligating the branch peptides to the template peptide and forming the template assembled synthetic protein (TASP).

9. A process for preparing a template assembled synthetic protein (TASP) as described in claim 8 wherein:
in said Step A, the haloacetyl functionality is a bromoacetyl functionality.

10. A process for preparing a template assembled synthetic protein (TASP) as described in claim 9 wherein:
in said Step A, the template side chain bearing the amino group bonded to the haloacetyl functionality is a lysine side chain corresponding to a lysine amino acid residue.

11. A process for preparing a template assembled synthetic protein (TASP) as described in claim 8 wherein:
in said Step C, the C-terminal end of the branch peptide being ligated to the amino acid residue of the template peptide by means of a linkage unit having the structure ψ(CO-S-CH₂-CO-NH) to form the template assembled synthetic protein (TASP).

12. A process for preparing a template assembled synthetic protein (TASP) as described in claim 8 wherein:
in said Step B, the branch peptides are pro-helical peptides which, upon incorporation into the template assembled synthetic protein (TASP) and exposure to helix promoting conditions, are capable of assuming a helical configuration; and then, after or concurrent with Step C,
Step D: exposing the formed template assembled synthetic protein (TASP) of said Step C to helix promoting conditions for promoting the formation of helices by the branch peptides.

## Patentansprüche

1. Vermittels eines Templats aufgebautes synthetisches Protein (TASP) mit einer dendritischen Verknüpfungseinheit mit der Struktur Ψ (CO-S-CH₂-CO-NH).

2. Vermittels eines Templats aufgebautes synthetisches Protein (TASP), wie in Anspruch 1 beschrieben, weiterhin umfassend:
vier dendritische Verknüpfungseinheiten mit der Struktur Ψ (CO-S-CH₂-CO-NH),
vier Abzweigpeptide, wobei jedes der Abzweigpeptide die Sequenz besitzt, und
ein Templat-Peptid mit der Sequenz ⁺NH₃-K(Ac-)-K(⁺NH₃)-K(Ac-)-P-G-K(Ac-)-E(COO-)-K(Ac-)-G-COO-,
wobei die vier Verknüpfungseinheiten die Abzweigpeptide an die vier K(Ac-)Reste des Templat-Peptids koppeln.

3. Verbessertes vermittels eines Templats aufgebautes synthetisches Protein (TASP) mit einem Abzweigpeptid mit einem C-terminalen Ende und einem Templat-Peptid einschliesslich eines Aminosäurerests mit einer eine Aminogruppe tragenden Templat-Seitenkette, wobei die Verbesserung Folgendes umfasst:
eine Verknüpfungseinheit mit der Struktur Ψ (CO-S-CH₂-CO-NH), welche das C-terminale Ende des Abzweigpeptids an das Templat-Peptid über die Aminogruppe der Templat-Seitenkette wie folgt koppelt: 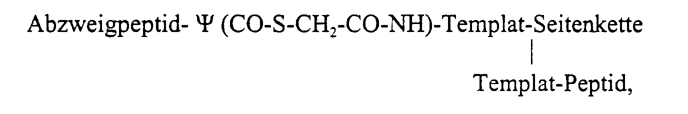 wobei das Abzweigpeptid als eine Verzweigung auf dem Templat-Peptid dient, um ein vermittels eines Templats aufgebautes synthetisches Protein (TASP) zu bilden.

4. Verbessertes vermittels eines Templats aufgebautes synthetisches Protein (TASP), wie in Anspruch 3 beschrieben, wobei:
die Templat-Seitenkette des Templat-Peptids eine Lysin-Seitenkette ist, die einem Lysin-Aminosäurerest entspricht.

5. Verbessertes vermittels eines Templats aufgebautes synthetisches Protein (TASP), wie in Anspruch 3 beschrieben, wobei:
das Abzweigpeptid ein prohelikales Peptid ist, welches nach Einbringung in das vermittels eines Templats aufgebaute synthetische Protein (TASP) und Exponierung an helixbildungsfördernde Bedingungen zur Annahme einer helikalen Konfiguration fähig ist.

6. Verbessertes, vermittels eines Templats aufgebautes synthetisches Protein (TASP), wie in Anspruch 3 beschrieben, wobei das Abzweigpeptid ein erstes Abzweigpeptid und ein zweites Abzweigpeptid einschliesst, jedes davon mit einem C-terminalen Ende, und das Templat-Peptid einen ersten Aminosäurerest mit einer ersten, eine Aminogruppe tragenden Templat-Seitenkette und einen zweiten Aminosäurerest mit einer eine Aminogruppe tragenden zweiten Templat-Seitenkette einschliesst, wobei die Verbesserung weiterhin umfasst:
die Verknüpfungseinheit einschliesslich einer ersten Verknüpfungseinheit und einer zweiten Verknüpfungseinheit, wobei jede Verknüpfungseinheit die Struktur Ψ (CO-S-CH₂-CO-NH) besitzt, die erste Verknüpfungseinheit zum Koppeln des C-terminalen Endes des ersten Abzweigpeptids an das Templat-Peptid über die erste Templat-Seitenkette dient und die zweite Verknüpfungseinheit zum Ankoppeln des C-terminalen Endes des zweiten Abzweigpeptids an das Templat-Peptid über die zweite Templat-Seitenkette dient,
wobei das erste und zweite Abzweigpeptid als erste und zweite Verzweigung auf dem Templat-Peptid dienen, um ein vermittels eines Templats aufgebautes synthetisches Protein (TASP) mit zwei Abzweigungen zu bilden.

7. Verbessertes, vermittels eines Templats aufgebautes synthetisches Protein (TASP), wie in Anspruch 6 beschrieben, wobei:
sowohl das erste als auch das zweite Abzweigpeptid prohelikale Peptide sind, die nach Einbringung in das vermittels eines Templats aufgebaute synthetische Protein (TASP) und Exponierung an helixbildungsfördernde Bedingungen zur Annahme helikaler Konfigurationen fähig sind.

8. Verfahren zur Herstellung eines vermittels eines Templats aufgebauten synthetischen Proteins (TASP), umfassend die folgenden Schritte:
Schritt A: Vorsehen eines Templat-Peptids einschliesslich eines Aminosäurerests mit einer Templat-Seitenkette, die eine an eine Halogenacetyl-Funktionalität gebundene Aminogruppe trägt;
Schritt B: Vorsehen eines oder mehrerer Abzweigpeptide(s), wobei jedes der Abzweigpeptide ein einen ^{α}COSH-Rest tragendes C-terminales Ende einschliesst; und danach
Schritt C: Kombinieren des Templat-Peptids aus Schritt A mit einem oder mehreren der Abzweigpeptide aus Schritt B unter nukleophilen Reaktionsbedingungen zur Förderung bzw. Beschleunigung einer Substitutionsreaktion zwischen dem ^{α}COSH-Rest der Abzweigpeptide und der Halogenacetyl-Funktionalität des Templat-Moleküls für das chemoselektive Ligieren der Abzweigpeptide mit dem Templat-Peptid und Bilden des vermittels eines Templats aufgebauten synthetischen Proteins (TASP).

9. Verfahren zur Herstellung eines vermittels eines Templats aufgebauten synthetischen Proteins (TASP), wie in Anspruch 8 beschrieben, wobei:
in Schritt A die Halogenacetyl-Funktionalität eine Bromacetyl-Funktionalität ist.

10. Verfahren zur Herstellung eines vermittels eines Templats aufgebauten synthetischen Proteins (TASP), wie in Anspruch 9 beschrieben, wobei:
in Schritt A die Templat-Seitenkette, die die an die Halogenacetyl-Funktionalität gebundene Aminogruppe trägt, eine Lysin-Seitenkette ist, die einem Lysin-Aminosäurerest entspricht.

11. Verfahren zur Herstellung eines vermittels eines Templats aufgebauten synthetischen Proteins (TASP), wie in Anspruch 8 beschrieben, wobei:
in Schritt C das C-terminale Ende des Abzweigpeptids mit dem Aminosäurerest des Templat-Peptids über eine Verknüpfungseinheit mit der Struktur Ψ (CO-S-CH₂-CO-NH) ligiert ist, um das vermittels eines Templats aufgebaute synthetische Protein (TASP) zu bilden.

12. Verfahren zur Herstellung eines vermittels eines Templats aufgebauten synthetischen Proteins (TASP), wie in Anspruch 8 beschrieben, wobei:
in Schritt B die Abzweigpeptide prohelikale Peptide sind, welche nach Einbringung in das vermittels eines Templats aufgebaute synthetische Protein (TASP) und Exponieren an helixbildungsfördernde Bedingungen zur Annahme einer helikalen Konfiguration fähig sind; und daran anschliessend, nach oder gleichzeitig mit Schritt C,
Schritt D: Exponieren des gebildeten, vermittels eines Templats aufgebauten synthetischen Proteins (TASP) von Schritt C an helixbildungsfördernde Bedingungen zur Förderung der Bildung von Helices durch die Abzweigpeptide.

## Revendications

1. Protéine synthétique assemblée sur matrice (TASP) ayant un motif de liaison dendritique de structure Ψ(CO-S-CH₂-CO-NH).

2. Protéine synthétique assemblée sur matrice (TASP) selon la revendication 1, comprenant en outre :
quatre motifs de liaison dendritique de structure Ψ(CO-S-CH₂-CO-NH),
quatre peptides de ramification, chacun desdits peptides de ramification ayant une séquence ⁺NH₃-D-A-A-T-A-L-A-N-A-L-K-K-L-G-COS-, et
un peptide matrice ayant une séquence ⁺NH₃-K(Ac-)-K(⁺NH₃)-K(Ac-)-P-G-K(Ac-)-E(COO⁻)-K(Ac-)-G-COO⁻,
les quatre motifs de liaison couplant lesdits peptides de ramification aux quatre résidus K(Ac-) dudit peptide matrice.

3. Protéine synthétique assemblée sur matrice (TASP) améliorée ayant un peptide de ramification pourvu d'une extrémité C-terminale et un peptide matrice comprenant un résidu amino acide avec une chaîne latérale matrice portant un groupe amino, protéine dans laquelle ladite amélioration comprend :
un motif de liaison ayant la structure Ψ(CO-S-CH₂-CO-NH) couplant l'extrémité C-terminale dudit peptide de ramification audit peptide matrice via le groupe amino de la chaîne latérale matrice comme suit :
grâce à quoi le peptide de ramification sert de ramification sur le peptide matrice pour former une protéine synthétique assemblée sur matrice (TASP).

4. Protéine synthétique assemblée sur matrice (TASP) améliorée selon la revendication 3, dans laquelle :
la chaîne latérale matrice dudit peptide matrice est une chaîne latérale lysine correspondant à un résidu amino acide de lysine.

5. Protéine synthétique assemblée sur matrice (TASP) améliorée selon la revendication 3, dans laquelle :
ledit peptide de ramification est un peptide pro-hélicoïdal qui, lors de son incorporation dans la protéine synthétique assemblée sur matrice (TASP) et l'exposition à des conditions favorisant la formation d'une hélice, est capable de prendre une configuration hélicoïdale.

6. Protéine synthétique assemblée sur matrice (TASP) améliorée selon la revendication 3, dans laquelle le peptide de ramification comprend un premier peptide de ramification et un second peptide de ramification, chacun avec une extrémité C-terminale, et le peptide matrice comprend un premier résidu amino acide avec une première chaîne latérale matrice portant un groupe amino et un second résidu amino acide avec une seconde chaîne latérale matrice portant un groupe amino, protéine dans laquelle ledit perfectionnement comprend en outre :
ladite unité de liaison comprenant une première unité de liaison et une seconde unité de liaison, chaque unité de liaison ayant la structure Ψ(CO-S-CH₂-CO-NH), la première unité de liaison servant à coupler l'extrémité C-terminale dudit premier peptide de ramification audit peptide matrice via la première chaîne latérale matrice et la seconde unité de liaison servant à coupler l'extrémité C-terminale dudit second peptide de ramification audit peptide matrice via la seconde chaîne latérale matrice,
grâce à quoi les premier et second peptides de ramification servent de première et seconde ramifications sur le peptide matrice pour former une protéine synthétique assemblée sur matrice (TASP) ayant deux ramifications.

7. Protéine synthétique assemblée sur matrice (TASP) améliorée selon la revendication 6, dans laquelle :
le premier peptide de ramification et le second peptide de ramification sont tous deux des peptides pro-hélicoïdaux qui, lors de leur incorporation dans la protéine synthétique assemblée sur matrice (TASP) et l'exposition à des conditions favorisant la formation d'une hélice, sont capables de prendre des configurations hélicoïdales.

8. Procédé de préparation d'une protéine synthétique assemblée sur matrice (TASP) comprenant les étapes suivantes :
étape A : la fourniture d'un peptide matrice comprenant un résidu amino acide ayant une chaîne latérale matrice portant un groupe amino lié à une fonctionnalité halogénoacétyle ;
étape B : la fourniture d'un ou plusieurs peptides de ramification, chacun desdits peptides de ramification comprenant une extrémité C-terminale portant un motif ^{α}COSH ; et
étape C : la combinaison du peptide matrice de l'étape A avec un ou plusieurs des peptides de ramification de l'étape B dans des conditions de réaction nucléophile pour promouvoir une réaction de substitution entre le motif ^{α}COSH des peptides de ramification et la fonctionnalité halogénoacétyle de la molécule matrice pour ligaturer chemo-sélectivement les peptides de ramification au peptide matrice et former la protéine synthétique assemblée sur matrice (TASP).

9. Procédé de préparation d'une protéine synthétique assemblée sur matrice (TASP) selon la revendication 8, dans lequel :
dans ladite étape A, la fonctionnalité halogénoacétyle est une fonctionnalité bromoacétyle.

10. Procédé de préparation d'une protéine synthétique assemblée sur matrice (TASP) selon la revendication 9, dans lequel :
dans ladite étape A, la chaîne latérale matrice portant le groupe amino lié à la fonctionnalité halogénoacétyle est une chaîne latérale lysine correspondant à un résidu amino acide de lysine.

11. Procédé de préparation d'une protéine synthétique assemblée sur matrice (TASP) selon la revendication 8, dans lequel :
dans ladite étape C, l'extrémité C-terminale du peptide de ramification est ligaturé au résidu amino acide du peptide matrice au moyen d'une unité de liaison ayant la structure Ψ(CO-S-CH₂-CO-NH) pour former la protéine synthétique assemblée sur matrice (TASP).

12. Procédé de préparation d'une protéine synthétique assemblée sur matrice (TASP) selon la revendication 8, dans lequel :
dans ladite étape B, les peptides de ramification sont des peptides pro-hélicoïdaux qui, lors de leur incorporation dans la protéine synthétique assemblée sur matrice (TASP) et leur exposition à des conditions favorisant la formation d'une hélice, sont capables de prendre une configuration hélicoïdale ; et ensuite, après ou concurremment avec l'étape C,
étape D : l'exposition de la protéine synthétique assemblée sur matrice (TASP) formée à ladite étape C à des conditions favorisant la formation d'une hélice pour favoriser la formation d'hélices par les peptides de ramification.


### SEQUENCE LISTING

(1) GENERAL INFORMATION:
   (i) APPLICANT:
      (A) NAME: The Scripps Research Institute
      (B) STREET: 10666 North Torrey Pines Road, TPC-8
      (C) CITY: La Jolla
      (D) STATE: CA
      (E) COUNTRY: USA
      (F) POSTAL CODE (ZIP): 92037
      (G) TELEPHONE: 619-554-2937
      (H) TELEFAX: 619-554-6312
   (ii) TITLE OF INVENTION: TEMPLATE ASSEMBLED SYNTHETIC PROTEIN
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: *Patent*In Release #1.0, Version #1.25 (EPO)
   (v) CURRENT APPLICATION DATE:
      (A) APPLICATION NUMBER: PCT/US/
      (B) FILING DATE: 11-AUG-1994
   (vi) PRIOR APPLICATION DATA:
      (A) APPLICATION NUMBER: US 08/105,369
      (B) FILING DATE: 11-AUG-1993
(2) INFORMATION FOR SEQ ID NO: 1:
   (i) SEQUENCE CHARACTERISTICS:
      (A) LENGTH: 14 amino acids
      (B) TYPE: amino acid
      (D) TOPOLOGY: linear
   (ii) MOLECULE TYPE: peptide
   (ix) FEATURE:
      (A) NAME/KEY: Thiolester-bond
      (B) LOCATION: 14
      (D) OTHER INFORMATION: /label= COS
         /note= ""COS indicates a thioester group on the
         C-terminal amino acid.""
   (xi) SEQUENCE DESCRIPTION: SEQ ID NO: 1:
   Asp Ala Ala Thr Ala Leu Ala Asn Ala Leu Lys Lys Leu Gly
   1               5                   10
(2) INFORMATION FOR SEQ ID NO: 2:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Ac
         /note= ""K(Ac) represents a lysine amino acid residue having an lysine side chain with an acetylated amino group.""
   (ix) FEATURE:
      (A) NAME/KEY: Modified-site
      (B) LOCATION: 8
      (D) OTHER INFORMATION: /label= Ac
         /note= ""K(Ac) represents a lysine amino acid residue having an lysine side chain with an acetylated amino group.""
   (xi) SEQUENCE DESCRIPTION: SEQ ID NO: 2:
   Lys Lys Lys Pro Gly Lys Glu Lys Gly
   1               5